# FASCICULE DE BREVET EUROPEEN

(11) **EP 3 932 468 B1**
(45) Date de publication et mention de la délivrance du brevet: **09.04.2025**
(21) Numéro de dépôt: 21171890.3
(22) Date de dépôt: 03.05.2021
(51) Int. Cl.: A61M 39/26, F16L 37/60, A61G 12/00, A61M 16/08, A61M 39/10

(54) **PRISE DE DISTRIBUTION DE FLUIDE PROTÉGÉE PAR UN BOITIER RIGIDE UTILISABLE EN MILIEU HOSPITALIER**
FLÜSSIGKEITSVERTEILER, GESCHÜTZT DURCH EINEN STARREN KASTEN FÜR DIE VERWENDUNG IN KRANKENHAUSEINSTELLUNGEN
FLUID DISTRIBUTION SOCKET PROTECTED BY A RIGID BOX FOR USE IN HOSPITAL SETTINGS

(30) Priorité: 29.06.2020 FR 2006793
(43) Date de publication de la demande: 05.01.2022
(73) Titulaire: Air Liquide Medical Systems, 92160 Antony (FR)
(72) Inventeur: CHEVEREAU, Christophe, 92160 Antony (FR); BEAUCHER, Jérôme, 92160 Antony (FR); RUDNIANYN, Philippe, 92160 Antony (FR); FAVRE REGUILLON, Loic, 92160 Antony (FR)
(74) Mandataire: Air Liquide

(56) Documents cités:
- FR-A- 437 827
- FR-A1- 3 086 174
- US-A- 4 123 089
- US-A- 5 020 563
- US-A1- 2007 215 210

## Description

L'invention concerne un ensemble de distribution de fluide mural comprenant une prise de distribution de fluide, notamment de distribution de gaz ou de vide, et un boitier rigide protégeant la prise, destiné à être fixé à une paroi au sein d'un bâtiment hospitalier ou analogue pour fournir du gaz ou du vide, i.e. aspiration/dépression.

Il est usuel d'utiliser des prises de distribution de fluide pour distribuer les fluides, en particulier les gaz médicaux (i.e. un gaz pur ou un mélange gazeux) ou le vide (i.e. dépression < 1 atm), au sein des bâtiments hospitaliers. On les trouve dans les chambres des patients, dans les salles d'opération ou de soins, ou d'autres pièces du bâtiment. Ces prises permettent de fournir les gaz médicaux véhiculés par les réseaux de canalisations de gaz parcourant les bâtiments hospitaliers, aux appareils et équipements utilisés pour traiter et soigner les patients au sein de ces bâtiments, en particulier les gaz thérapeutiques, tels l'oxygène, le protoxyde d'azote ou l'air, ou le vide médical (i.e. aspiration/dépression) permettant d'opérer des aspirations notamment de liquides biologiques, par exemple le sang ou d'autres liquides biologiques.

Les prises de distribution de fluide sont aussi appelées « prises murales » ou « raccords muraux », car elles sont généralement montées soit directement sur les parois, c'est-à-dire les murs, cloisons ou analogues, des bâtiments hospitaliers, soit indirectement, par exemple en étant intégrées à un boitier ou analogue qui est lui-même monté sur une paroi. Elles peuvent être aussi montées dans les "gaines tête de lit" utilisées dans les chambres au chevet des patients ou dans les "bras plafonniers", utilisés dans les blocs opératoires.

Le document FR-A-2628820 propose une prise de distribution de fluide de ce type, appelée raccord à verrouillage automatique, comprenant un corps de prise de forme allongée comprenant un passage axial traversant axialement le corps de prise de sorte de relier fluidiquement une extrémité amont à une extrémité aval. Le fluide « circule » d'une extrémité à l'autre, lorsqu'un connecteur d'un appareil ou équipement médical, telle connecteur d'une conduite de fluide, est raccordé mécaniquement et fluidiquement à la prise. Le corps de prise comprend aussi une tubulure, c'est-à-dire un petit conduit, en communication fluidique avec le passage axial traversant le corps de prise et qui est par ailleurs solidarisée au corps de prise selon un axe perpendiculaire à l'axe du corps de prise. Cette tubulure vient se raccorder au réseau de fluide, c'est-à-dire de gaz ou de vide, de l'hôpital. Lorsque la prise murale est agencée sur une paroi, la tubulure doit être verticale, alors que le passage axial du corps de prise doit être horizontal.

Une prise murale est souvent insérée dans un boitier rigide qui sert à la protéger notamment contre les chocs et les salissures, notamment les dépôts de poussières ou autres.

Afin d'aider les soignants à sélectionner la prise murale souhaitée, lorsque plusieurs prises murales sont agencées sur une même paroi, les unes à côté des autres, le nom du fluide, par exemple « oxygène », « protoxyde d'azote », « air médical » ou « vide » est en général indiqué sur le boitier.

Or, certains utilisateurs non-autorisés, par exemple un peintre ayant à repeindre le mur sur lequel sont fixées plusieurs prises murales, démontent les boitiers mais sans penser à repérer quel boitier correspond à quelle prise. Lors du remontage, il peut alors se produire des inversions de boitiers, conduisant à des situations à risques pour les patients, par exemple si un boitier avec une indication « oxygène » est monté par erreur sur une prise murale fournissant du protoxyde d'azote.

Par exemple, les documents US2007/215210, US4123089, FR437827 et US5020563 proposent des systèmes de distribution de gaz dont les éléments constitutifs sont assemblés au moyen de vis ou analogues.

Le problème est donc de pouvoir éviter ou minimiser le risque qu'un boitier de prise murale ne puisse être démonté facilement par une personne non-autorisée, c'est-à-dire autre qu'un opérateur de maintenance qualifié pour démonter et entretenir les prises murales et leurs boitiers.

La solution de l'invention porte sur un ensemble de distribution de fluide, i.e. de gaz ou de vide, destiné à être fixé à une paroi d'un bâtiment hospitalier comprenant une prise de distribution de fluide comprenant un corps de prise et un boitier agencé autour du corps de prise de la prise de distribution de fluide, ledit boitier comprenant (au moins) un demi-boitier avant et une façade avant comprenant une ouverture centrale donnant accès à la prise de distribution de fluide, et une pièce-écrou comprenant un orifice central et une face extérieure en couronne orientée vers l'extérieur du boitier, ladite pièce-écrou étant fixée au corps de prise de sorte que la façade avant soit prise en sandwich et maintenue entre la pièce-écrou et le corps de prise.

L'ensemble de distribution de fluide selon l'invention est caractérisé en ce que:
- le demi-boitier avant formant le corps principal du boitier rigide est situé entre la face avant et une pièce-étrier, ledit demi boitier étant pris en 'sandwich' entre la face avant et la paroi du bâtiment hospitalier, et ledit corps de prise étant maintenu sur la paroi du bâtiment hospitalier, par la pièce-étrier,
- plusieurs logements sont agencés dans la face extérieure en couronne de la pièce-écrou, lesdits logements étant dimensionnés (i.e. configurés) pour recevoir un outil spécifique de montage/démontage de la pièce-écrou,
- la pièce-écrou comprend en outre un orifice central comprenant un taraudage aménagé dans la paroi périphérique délimitant l'orifice central, ledit taraudage de la pièce-écrou coopérant avec un filetage périphérique porté par le corps de prise de la prise de distribution de fluide pour fixer par vissage la pièce-écrou audit corps de prise au moyen dudit outil spécifique de montage/démontage, et
- la prise de distribution de fluide comporte en outre un guide-embout agencé coaxialement dans le corps de prise, ledit guide-embout comprenant une tête de raccordement faisant saillie axialement vers l'extérieur au travers de l'ouverture centrale de la façade du boitier, l'orifice central de la pièce-écrou étant traversé par la tête de raccordement du guide-embout.

Selon le mode de réalisation considéré, l'ensemble de distribution de fluide de l'invention peut comprendre l'une ou plusieurs des caractéristiques suivantes :
- les logements aménagés dans la face extérieure en couronne de la pièce-écrou sont dimensionnés pour recevoir une clé de montage/démontage en tant qu'outil spécifique de montage/démontage.
- les logements aménagés dans la face extérieure en couronne de la pièce-écrou sont borgnes, c'est-à-dire qu'ils ne traversent pas complètement l'épaisseur de la pièce-écrou.
- il comprend au moins deux logements diamétralement opposés aménagés dans la face extérieure en couronne de la pièce-écrou.
- il comprend de 2 à 10 logements aménagés dans la face extérieure en couronne de la pièce-écrou.
- les logements aménagés dans la face extérieure en couronne de la pièce-écrou sont des trous ou perçages borgnes.
- l'orifice central de la pièce-écrou, le corps de prise et l'ouverture centrale de la façade avant sont coaxiaux (selon un axe AA).
- la pièce-écrou a un périmètre circulaire.
- la pièce-écrou a un diamètre extérieur (D) compris entre 26 et 50 mm, typiquement entre 30 et 45 mm.
- la pièce-écrou comprend un orifice central comprenant un taraudage aménagé dans l'épaisseur de la pièce-écrou au sein de l'orifice central.
- il comprend en outre un outil spécifique de montage/démontage, telle une clé spécifique de montage/démontage, comprenant des griffes aptes à venir s'insérer dans les logements de la pièce-écrou afin de permettre à un opérateur d'exercer un couple dans le sens horaire ou antihoraire afin de visser ou dévisser la pièce-écrou.
- une rainure annulaire, c'est-à-dire un évidement ou une gorge, est aménagée autour de l'ouverture centrale de la façade du boitier.
- la rainure annulaire définit avec l'ouverture centrale de la façade du boitier une zone en retrait, de préférence en forme de disque, c'est-à-dire cylindrique.
- la rainure annulaire, i.e. évidement ou gorge, est ouverte sur l'ouverture centrale de la façade du boitier.
- la zone en retrait est dimensionnée pour loger la pièce-écrou.
- la zone en retrait a une forme complémentaire de la forme de la pièce-écrou, i.e. la zone en retrait est dimensionnée pour recevoir et loger la pièce-écrou, notamment une forme de disque ou cylindrique.
- le contour circulaire externe (i.e. périphérie) de la pièce-écrou épouse la forme circulaire de la périphérie interne de la zone en retrait.
- la pièce-écrou vient se loger dans la zone en retrait définit par la rainure annulaire et l'ouverture centrale de la façade du boitier lorsque la pièce-écrou maintient la façade avant au contact du corps de prise, c'est-à-dire lorsque la pièce-écrou est vissée au corps de prise en prenant la façade avant en sandwich.
- le boitier a une forme parallélépipédique.
- le boitier comprend le demi-boitier avant et un demi-boitier arrière fixés l'un à l'autre.
- le boitier comprend en outre un couvercle pivotant porté par la façade avant.
- une tubulure verticale est connectée fluidiquement au corps de la prise.
- le boitier comprend en outre une ouverture de toit, située sur le dessus du boitier, traversée par la tubulure verticale connectée fluidiquement au corps de la prise.
- la tubulure verticale est raccordé fluidiquement au réseau de fluide d'un bâtiment hospitalier.
- la pièce-écrou est en métal ou alliage métallique, par exemple en laiton ou en acier.
- la pièce-écrou a une épaisseur comprise entre 2 et 8 mm.
- la pièce-écrou a une forme de disque percé en son centre.
- la pièce-écrou comprend un orifice central a un diamètre intérieur (d) compris entre 20 et 30 mm.
- les griffes de l'outil spécifique de montage/démontage, e.g. une clé spécifique de montage/démontage, sont des picots, protubérances, têtes en relief ou analogues, configurées et/ou dimensionnées pour venir s'insérer dans les logements de la pièce-écrou afin de permettre à un opérateur d'exercer un couple dans le sens horaire ou antihoraire afin de visser ou dévisser la pièce-écrou.
- la prise de distribution de fluide comprend un corps de prise de forme allongée selon un axe (AA), comprenant un passage central traversant axialement (AA) le corps de prise.
- une gorge annulaire aménagée est dans la paroi périphérique du corps de prise.
- un filetage périphérique est aménagé est dans la paroi périphérique du corps de prise. Ce filetage périphérique porté par le corps de prise coopère avec le taraudage de la pièce-écrou pour fixer par vissage la pièce-écrou audit corps de prise.
- le corps de prise comprend une tubulure, c'est-à-dire un petit conduit, en communication fluidique avec le passage central du corps de prise, la tubulure étant solidarisée au corps de prise selon un axe (BB) perpendiculairement à l'axe (AA) du corps de prise.
- une pièce-étrier comprenant une ouverture bordée par un rebord arqué vient se loger dans la gorge annulaire du corps de prise afin de fixer le corps de prise à une cloison.
- la pièce-étrier comprend en outre des perçages traversant la pièce-étrier, de préférence au moins l'un desdits perçages étant de forme circulaire et au moins l'un desdits perçages étant de forme oblongue, de préférence la pièce-étrier comprenant trois perçages traversant la pièce-étrier, typiquement deux perçages sont de forme oblongue.
- les perçages sont dimensionnés pour recevoir des moyens d'arrimage par vissage, de préférence des vis, des boulons ou analogues.
- les perçages permettent de palier l'hyperstatisme de l'assemblage.
- la tubulure du corps de prise est rigide.
- le corps de prise est rigide.
- la tubulure est en métal ou alliage métallique.
- le corps de prise est en métal ou alliage métallique.
- le métal ou alliage métallique formant la tubulure et/ou le corps de prise est choisi parmi le cuivre, le laiton et l'acier.
- la tubulure forme tout ou partie de moyens de raccordement permettant de raccorder mécaniquement et fluidiquement une canalisation de fluide, en particulier une canalisation de fluide faisant partie d'un réseau de canalisations de fluide (i.e. gaz ou vide) agencé au sein d'un bâtiment hospitalier de sorte que ledit corps de prise soit en communication fluidique avec ladite canalisation de fluide.
- la tubulure comprend un lumen dans lequel circule le fluide, i.e. gaz ou dépression.
- la tubulure est fixée au corps de prise par soudage ou autre.
- le rebord arqué de la pièce-étrier a une forme complémentaire de la forme de la gorge annulaire du corps de prise.
- l'ouverture de la pièce-étrier a une section (sensiblement) circulaire, c'est-à-dire en cercle ou partie de cercle.
- la pièce-étrier est une plaque d'environ 0,5 mm à 5 mm d'épaisseur au travers de laquelle sont percés des perçages de fixation et une découpe arrondie constituant l'ouverture bordée par le rebord arqué, de préférence de 2 à 3 mm d'épaisseur.
- le boitier rigide agencé autour du corps de prise est un boitier de protection servant notamment à protéger la prise contre les poussières et les chocs éventuels.
- la prise comporte un guide-embout agencé coaxialement dans le corps de prise.
- le guide-embout est monté extractible, c'est-à-dire démontable, dans le corps de prise.
- le guide-embout comprend un logement axial comprenant un clapet de tête mobile au sein dudit logement axial, un siège de clapet et un élément élastique.
- le clapet de tête est normalement repoussé contre le siège de clapet par l'élément élastique.
- le guide-embout extractible comprend une gorge annulaire aménagée dans sa paroi périphérique externe et un premier joint d'étanchéité annulaire agencé dans ladite gorge annulaire.
- le guide-embout est en un matériau rigide choisi parmi les alliages de cuivre, en particulier le laiton nickelé, et les alliages de zinc, d'aluminium et de magnésium, en particulier le zamak.
- le clapet de tête est mobile axialement (i.e. selon l'axe AA) au sein du logement axial, de préférence mobile en translation, en particulier coulissant.
- le clapet de tête, le siège de clapet et l'élément élastique forment tout ou partie d'éléments de contrôle du passage de gaz.
- l'élément élastique comprend un ressort ou analogue, par exemple un ressort métallique de forme cylindrique ou tronconique.
- l'élément élastique forme manchon autour d'au moins une partie du clapet de tête.
- un second joint d'étanchéité, tel un joint torique, est agencé autour du clapet de tête.
- le second joint d'étanchéité coopère avec le siège de clapet pour assurer une étanchéité fluidique entre clapet de tête et siège de clapet, lorsque le clapet de tête est totalement repoussé contre le siège de clapet par l'élément élastique.
- le premier et/ou le second joint d'étanchéité est en élastomère, en silicone ou autre.
- le guide-embout est fixé par vissage dans le corps de prise.
- le clapet de tête comprend un logement interne borgne comprenant un orifice axial et un ou plusieurs orifices latéraux en communication fluidique avec le logement interne borgne, de préférence au moins 3 ou 4 orifices latéraux.
- les orifices latéraux sont agencés en couronne autour du clapet de tête
- le guide-embout forme manchon autour du clapet de tête.
- le guide-embout est formé d'au moins deux sous-unités fixées l'une à l'autre.
- le guide-embout comprend un logement axial se terminant par un orifice proximal.
- l'orifice axial du clapet de tête et l'orifice proximal du guide-embout sont agencés coaxiaux.
- le clapet de tête a (au moins en partie) une forme générale tubulaire.
- le clapet de tête comprend une portion avant tubulaire au sein de laquelle est aménagée le logement interne borgne.
- les orifices latéraux sont percés au travers de la paroi latérale de la portion avant tubulaire.
- le passage central du corps de prise relie fluidiquement une extrémité distale à une extrémité proximale.
- le guide-embout est monté de manière extractible, c'est-à-dire qu'il peut être extrait ou démonté de la prise.
- l'élément élastique est agencé autour d'au moins une partie de la partie distale (ou partie arrière) du clapet de tête.
- l'élément élastique vient appuyer, en particulier par l'une de ses extrémités, sur un épaulement agencé autour du clapet de tête et solidaire de celui-ci, notamment un épaulement de forme annulaire.
- le guide-embout comprend un fond, de préférence un fond borgne, sur lequel vient appuyer, en particulier par l'autre de ses extrémités, l'élément élastique.
- un système de sécurité, agencé dans le passage central, comprend une bille-clapet ou analogue et une chambre à bille dans laquelle est logée la bille-clapet, et un siège de clapet à bille coopérant avec ladite bille-clapet pour contrôler le passage de fluide.
- le guide-embout comprend une expansion venant coopérer avec la bille-clapet du système de sécurité.
- le boitier rigide comprend une ouverture sur le dessus permettant de laisser passer la tubulure de la prise, en position verticale.
- le boitier rigide comprend un compartiment interne comprenant la prise de distribution de fluide, et une face avant portant un couvercle pivotant donnant accès audit compartiment interne.
- l'orifice central de la façade avant du boitier est traversé par une partie de la prise de distribution de fluide.
- le boitier rigide a une forme générale parallélépipédique rectangle.

L'invention porte aussi sur une utilisation d'un ensemble selon l'invention pour distribuer un fluide, typiquement un gaz médical ou du vide, au sein d'un bâtiment hospitalier, le boitier dudit ensemble étant fixé (i.e. directement ou indirectement) à une cloison dudit bâtiment hospitalier.

De préférence, la prise de distribution de fluide (i.e. gaz ou vide) et/ou l'ensemble de distribution de fluide mural selon l'invention sont fixés à la paroi par des moyens à vis et de la pièce-étrier.

L'invention va maintenant être mieux comprise grâce à la description détaillée suivante, faite à titre illustratif mais non limitatif, en référence aux figures annexées parmi lesquelles :
Fig. 1 est une vue éclatée d'un mode de réalisation d'un ensemble de distribution de fluide comprenant une prise murale et son boitier conforme à la présente invention ;
Fig. 2 est une vue associée de l'ensemble de la Fig. 1 ;
Fig. 3 représente un mode de réalisation de la pièce-écrou servant à la fixation du boitier à la prise murale d'un ensemble de distribution de fluide selon la présente invention ;
Fig. 4 représente un autre mode de réalisation de la pièce-écrou servant à la fixation du boitier à la prise murale d'un ensemble de distribution de fluide selon la présente invention; et
Fig. 5 est un schéma en coupe illustrant l'imbrication de la pièce-écrou dans une zone en retrait de la façade du boitier.

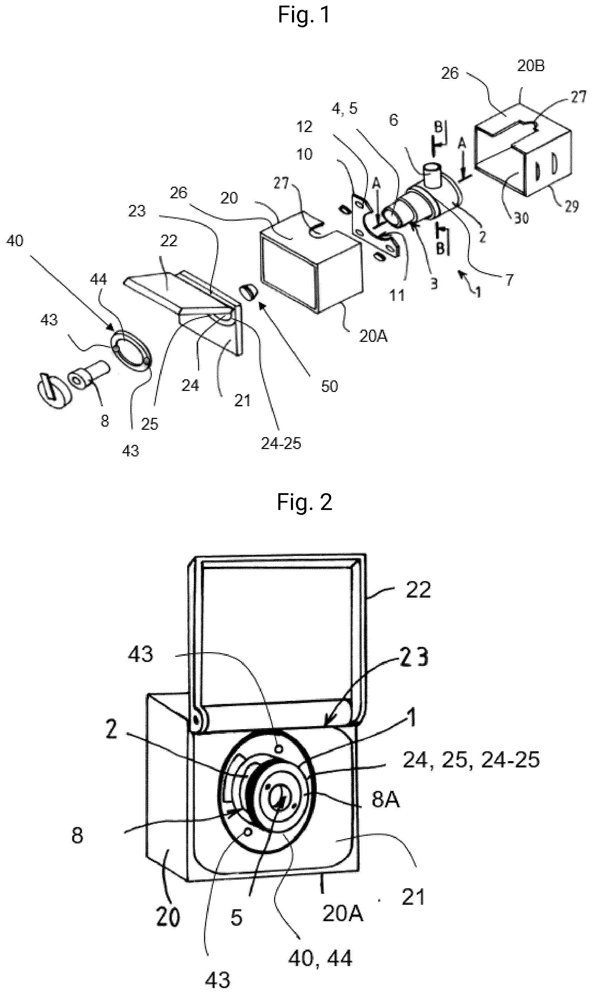

Fig. 1 est une vue éclatée d'un mode de réalisation d'un ensemble de distribution de fluide 1 comprenant une prise de distribution 2 de fluide, appelée simplement prise murale 2 ci-après, et son boitier 20 conforme à la présente invention.

La prise de distribution de fluide ou prise murale 2 comprend un corps de prise 3 de forme allongée, selon un axe longitudinal AA, à savoir un axe horizontal lorsque la prise murale 2 est fixée à une paroi verticale (i.e. mur, cloison...) d'un bâtiment hospitalier, par exemple un mur vertical.

Le corps de prise 3 est traversé axialement (selon axe AA) par un passage axial 4 pour le fluide (i.e. gaz ou vide) formant un logement interne au sein duquel sont agencés, de manière extractible, des éléments de contrôle du passage de gaz.

Classiquement, les éléments de contrôle du passage de gaz comprennent un guide-embout et un système de sécurité de type chambre à bille, qui sont montés extractibles dans le corps de prise 3.

Plus précisément, le guide-embout 8, représenté extrait du corps de prise 3 sur Fig. 1 et inséré dans le corps de prise 3 sur Fig. 1, comprend un logement axial, un clapet de tête mobile axialement, i.e. coulissant en translation, au sein dudit logement axial et un orifice proximal de passage de fluide, i.e. gaz ou vide, lequel orifice est de section circulaire. Par exemple, lorsque le fluide est un gaz (i.e. gaz pur ou mélange gazeux), celui-ci est distribué, c'est-à-dire sort du corps 3 de la prise 2, par l'orifice proximal 5. Est également prévu un élément élastique, tel un ressort cylindrique ou analogue, agencé dans le logement axial du guide-embout 8 et permettant de normalement repousser le clapet de tête contre un siège de clapet aménagé dans le guide-embout 8 de sorte de contrôler le passage de fluide dans le corps de prise.

Par ailleurs, le système de sécurité est agencé dans le passage axial et comprend une bille-clapet ou analogue (i.e. demi-sphère ou sphère) et une chambre à bille 50 dans laquelle est logée la bille-clapet, et un siège de clapet à bille coopérant avec ladite bille-clapet pour contrôler le passage de fluide. Dans le cas d'une prise de vide, la bille-clapet est préférentiellement une demi-sphère coopérant avec un ressort de rappel.

La chambre à bille 50 est montée extractible dans le corps 3 de prise. Elle est située entre le fond de prise et le guide-embout 8, qui est lui-même démontable.

Ce système de sécurité à chambre à bille 50 permet d'empêcher que le gaz ne puisse s'échapper de la prise 2 ou que l'aspiration par le vide (i.e. dépression) ne se fasse, c'est-à-dire que de l'air ne puisse entrer dans le réseau de vide relié à la prise murale 2 et y faire remonter la pression (< 1 atm) qui y règne, lorsque le guide-embout 8 est démonté et extrait du corps de prise 3, notamment lors d'une opération de vérification, de maintenance ou de remplacement. La bille-clapet fait office de clapet de sécurité venant, sous l'effet de la pression fluidique ou de la dépression (i.e. vide) s'exerçant sur celle-ci, appuyer sur et obturer le siège de clapet de la chambre à bille 50, lorsque le guide-embout 8 est extrait du corps 3 de la prise murale 2, de sorte d'interrompre toute liaison fluidique.

A l'inverse, quand le guide-embout 8 est monté dans le corps de prise 3, il vient appuyer sur la bille-clapet emprisonnée dans la chambre à bille 50 pour la décoller du siège de clapet et autoriser ainsi la communication fluidique au travers de l'orifice ou canal de liaison. Ceci peut se faire via une tige axiale, formant une expansion ou analogue, faisant saillie sur la surface externe arrière du guide-embout 8 et solidaire de celui-ci, qui est orientée de sorte de traverser axialement l'orifice ou canal de liaison pour venir appuyer sur la bille-clapet et ainsi la décoller du siège de clapet.

Il est à noter que la bille-clapet peut avoir différentes formes, notamment une forme de sphère, de demi-sphère ou une autre forme adaptée, par exemple ovale, ovoïde ou autre.

De même, le guide-embout 8 peut être formé de plusieurs sous-unités fixées l'une à l'autre, par exemple par vissage, emboitement, soudage ou autre, et peut être réalisé en métal ou alliage métallique, en particulier, en alliage de cuivre, tel le laiton, de préférence nickelé, i. e. laiton nickelé, ou en alliage de zinc, d'aluminium et de magnésium, en particulier le zamak, ou en polymère.

Une telle architecture de prise murale 2 est connue, notamment de FR-A-2628820.

Le raccordement fluidique de la prise murale 2 au réseau de canalisations de fluide se fait via une tubulure 6, c'est-à-dire un petit conduit de gaz, qui est en communication fluidique, via son lumen, avec, d'une part, ledit réseau de gaz ou de vide et, d'autre part, avec le passage central interne de la prise murale 2. La tubulure 6 est solidarisée au corps de prise 3 selon un axe (BB) perpendiculairement à l'axe (AA) du corps de prise 3, comme illustré en Fig. 1, par exemple fixée par soudage ou autre. La tubulure 6 et le corps de prise 3 sont rigides, de préférence en métal ou alliage métallique, par exemple en laiton, en cuivre ou en acier. Ainsi, lorsque la prise murale 1 est fixée à une paroi, l'axe (BB) de la tubulure 6 est sensiblement vertical et l'axe (AA) du corps de prise 3 est sensiblement horizontal.

Afin de faciliter la fixation et surtout l'indexage angulaire du corps 3 de la prise murale 2 de manière à ce que la tubulure 6 faisant la jonction entre le réseau de gaz ou de vide de l'hôpital et le passage axial du corps 3 de la prise 2, soit verticale ou quasi-verticale, il est prévu une gorge annulaire 7 aménagée dans la paroi périphérique cylindrique du corps 3 de la prise murale 2, et une pièce-étrier 10 comprenant une ouverture 11 bordée par un rebord arqué, i.e. une découpe arrondie, venant se loger dans la gorge annulaire 7 du corps de prise 3, lorsque le corps de prise 3 est positionné dans la pièce-étrier 10, comme illustré en Fig. 1.

La pièce-étrier 10 comprend des moyens de fixation à une paroi, par exemple des perçages 12 destinés à recevoir des vis, des boulons ou tout autre élément ou moyen d'arrimage par vissage. Ici, la pièce-étrier 10 a une forme générale en « L » et comprend 3 perçages 12, par exemple un perçage de forme circulaire et deux perçages de forme oblongue, par exemple sensiblement ovale ou ellipsoïdale, situés aux extrémités du « L » de manière à pallier l'hyperstatisme du montage résultant du centrage de l'étrier sur le corps de prise et de la fixation de l'étrier au mur par 3 vis ou analogue passant par les perçages. Par exemple, la pièce-étrier 10 peut être réalisée dans une plaque d'environ 0,5 mm à 5 mm d'épaisseur, au travers de laquelle sont percés les perçages 12 de fixation et la découpe arrondie, i.e. en demi-cercle, constituant l'ouverture 11 bordée par le rebord arqué qui a une forme complémentaire de celle de la gorge annulaire du corps de prise 3.

Grâce à un tel agencement, il est extrêmement aisé et rapide de non seulement fixer le corps de prise 3 à la pièce-étrier 10 mais aussi d'indexer ces deux parties l'une par rapport à l'autre de manière à ce que la tubulure 6 soit verticale. Ceci est opéré par un opérateur, lors du montage de la prise murale 2 sur une paroi verticale, tel un mur, via une simple orientation selon un mouvement de rotation horaire ou antihoraire du corps 3 dans la pièce-étrier 10 jusqu'à mettre la tubulure 6 en position désirée, i.e. verticale. Une fois le corps 3 de la prise murale 2 correctement positionné et indexé dans la pièce-étrier 10, il suffit que l'opérateur agissent sur les moyens de fixation, notamment opère un serrage des vis, boulons ou autres éléments ou moyens d'arrimage par vissage passant au travers des perçages 12 et venant s'arrimer à la paroi pour solidariser fermement la prise 2 à la paroi verticale.

Par ailleurs, pour la protéger contre les chocs et des dépôts de poussières notamment, la prise murale 2 est agencée dans un boitier rigide 20 périphérique définissant un compartiment interne 30 pour recevoir la prise murale 2 et la protéger, comme illustré en Fig. 1 et Fig. 2.

Le boitier 20 rigide est par exemple parallélépipédique. Il peut être formé de plusieurs parties ou sous-unités assemblées les unes aux autres, à savoir ici deux demi-boitiers avant et arrière 20A, 20B, ici parallélépipédiques, venant s'emboiter l'un dans l'autre, ainsi qu'une face avant ou façade 21 portant un couvercle ou capot pivotant 22 autour d'une charnière 23, donnant accès à la prise 2 située dans le boitier 20. Les demi-boitiers avant et arrière 20A, 20B comprennent en outre des découpes 27 dans leur paroi de toit 26, permettant le passage de la tubulure 6, comme illustré en Fig. 1.

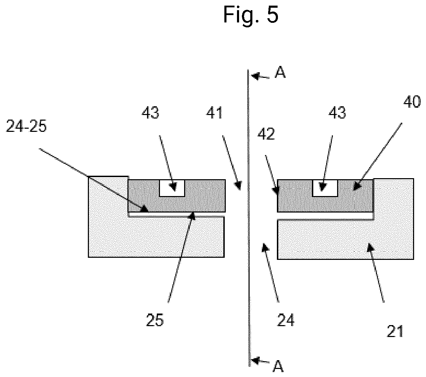

La façade avant 21 comprend une ouverture centrale 24 traversante, c'est-à-dire s'étendant au travers de l'épaisseur de la façade avant 21 entre les deux faces opposées de la façade avant 21, à savoir entre sa face extérieure 44 orientée vers l'extérieure et sa face intérieure orientée vers l'intérieur du boitier 20. Cette ouverture centrale 24 est bordée extérieurement par une rainure ou évidement 25 annulaire aménagée autour de ladite ouverture centrale 24, c'est-à-dire dans la région périphérique et adjacente de l'ouverture centrale 24, comme expliqué ci-après et schématisé en Fig. 5.

Comme illustré en Fig. 2, le guide-embout 8 comprend une tête de raccordement 8A, en général en métal, faisant saillie axialement vers l'extérieur, au travers de l'ouverture centrale 24 de la façade 21 du boitier 20, et à laquelle on peut raccorder mécaniquement et fluidiquement un connecteur d'un appareil ou équipement médical, par exemple le connecteur d'une conduite de fluide flexible.

Il est à noter que le demi-boitier arrière 20B, c'est-à-dire la sous-unité ou partie arrière du boitier, est optionnel et peut être omis dans certains cas, par exemple en cas d'intégration ou d'encastrement de l'ensemble 1 dans un logement percé dans le mur devant porter l'ensemble 1 boitier/prise murale. Toutefois, lorsque le demi-boitier arrière 20B est présent, comme sur la Fig. 1, sa paroi arrière est percée de trous afin de permettre le passage des vis ou analogue (non montré) de fixation qui traversent les perçages 12 de la pièce-étrier 10.

Le demi-boitier avant 20A forme le corps principal du boitier rigide 20. Il est en quelque sorte situé entre la pièce-étrier 10 et la face avant 21 portant le couvercle pivotant 22. Plus précisément, le demi boitier 20A est sensiblement pris en 'sandwich' entre la face avant 21 et le support d'appui de la face arrière du corps de prise 3, typiquement un mur, une paroi ou analogue. Le corps de prise 3 est lui-même maintenu sur le support d'appui, e.g. mur ou analogue, par la pièce étrier 10.

Conformément à la présente invention, afin de maintenir des éléments de la prise murale 2 solidaires les uns des autres, il est prévu une pièce-écrou 40 ayant une structure particulière venant coopérer avec le corps de prise 3.

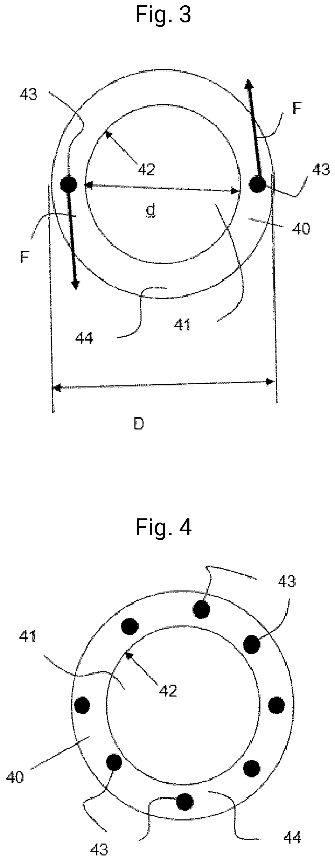

Plus précisément, comme mieux visible sur les Fig. 3 et Fig. 4, la pièce-écrou 40 a une forme de disque et comprenant un orifice central 41 traversant, c'est-à-dire que la pièce-écrou 40 est percée en son centre de manière à former une couronne ou un anneau aplati.

Un taraudage 42 est aménagé dans la paroi périphérique délimitant l'orifice central 41, c'est-à-dire dans l'épaisseur de la pièce-écrou 40 au sein de l'orifice central 41. Le taraudage 42 permet de fixer la pièce-écrou 40 par vissage autour du corps de prise 3, lequel porte un filetage complémentaire autour de sa périphérie externe.

Typiquement, la pièce-écrou 40 a une épaisseur de quelques millimètres. La périphérie de la pièce-écrou 40 est circulaire et de préférence totalement lisse, c'est-à-dire sans aspérités, crantages, logements ou autres.

Par ailleurs, selon l'invention, plusieurs logements 43 sont aménagés dans l'une 44 des surfaces ou faces de la pièce-écrou 40, à savoir la face extérieure 44 en couronne, qui est orientée vers l'extérieur du boitier 20, comme visible sur la Fig. 2. Ces logements 43 permettent de recevoir un outil spécifique de montage/démontage de la pièce-écrou 40, comme expliqué ci-après.

Autrement, ces logements 43, qui sont préférentiellement borgnes, sont dimensionnés et/ou configurés pour recevoir un outil spécifique de montage et/ou démontage servant à visser ou dévisser la pièce-écrou 40 afin de la fixer ou, à l'inverse, de la démonter du corps de prise 3.

L'orifice central 41 de la pièce-écrou 40 est traversé par la tête de raccordement 8A du guide-embout 8, laquelle fait saillie axialement vers l'extérieur, au centre de la façade 21 du boitier 20, c'est-à-dire qu'elle se projette axialement vers l'extérieur au-delà de la surface externe de la façade 21 du boitier 20, comme illustré en Fig. 2, afin de permettre sa connexion ou raccordement fluidique à un dispositif ou appareil médical.

Par ailleurs, comme susmentionné, une rainure ou évidement 25 annulaire est aménagé dans la face extérieure 44 en couronne de la façade 21 du boitier 20 orientée vers l'extérieur du boitier 20. Cette rainure annulaire 25 est formée dans la région périphérique immédiatement adjacente à l'ouverture centrale 24 de la façade 21 du boitier 20 de manière à définir avec cette ouverture centrale 24, une zone en retrait 24-25 en forme de disque au sein de laquelle la pièce-écrou 40 vient se loger, comme illustré en Fig. 2 et Fig. 5.

Autrement dit, la rainure ou évidement 25 annulaire forme avec l'ouverture centrale 24 de la façade 21, une zone en retrait 24-25 ou empreinte de forme complémentaire de celle de la pièce-écrou 40, typiquement une zone en retrait 24-25 (i.e. un logement) en forme de disque, dans laquelle vient se positionner la pièce-écrou 40, lorsqu'elle est vissée au corps 3 de prise, via le taraudage 42. L'insertion de la pièce-écrou 40 coaxiale (AA) au sein de la zone en retrait 24-25 ou empreinte de forme complémentaire de celle de la pièce-écrou 40 est illustré en Fig. 5.

Les logements 43, typiquement à fond borgne, portés par la zone en couronne de de la pièce-écrou 40 servent au montage et démontage de la pièce-écrou 40, donc au désassemblage du boitier 20 au moyen d'un outil spécifique, telle une clé de montage/démontage. Autrement dit, les logements 43 ne sont pas traversés par des vis ou analogues.

L'outil spécifique de montage/démontage comprend des griffes ou analogues venant s'insérer dans les logements 43 afin de permettre à un opérateur d'y exercer un couple, c'est-à-dire une force, dans le sens horaire ou antihoraire permettant de visser ou dévisser la pièce-écrou 40. Les flèches F orientées dans le sens antihoraire sur la Fig. 3 illustrent un dévissage, c'est-à-dire un démontage, de la pièce-écrou 40. Le serrage se fait dans le sens opposé.

Le mode de réalisation de la Fig. 3 présente 2 logements 43 borgnes diamétralement opposés agencés autour de l'orifice central 41 de la pièce-écrou 40, alors que celui de la Fig. 4 montre 8 logements 43 répartis sur la couronne 44 bordant l'orifice central 41 de la pièce-écrou 40.

De façon générale, les logements 43 permettent d'empêcher n'importe quelle personne non-habilitée, ne possédant par l'outil spécifique de montage/démontage, de démonter la pièce-écrou 40, ce qui minimise les risques et problèmes susmentionnés, qui peuvent exister notamment lorsque de simples vis sont utilisées pour assembler ces éléments.

Une prise murale 1 selon l'invention est conçue pour fournir soit des gaz thérapeutiques, tels l'oxygène, le protoxyde d'azote, l'air ou tout autre gaz ou mélange gazeux, soit du vide médical (i.e. dépression) servant à opérer des aspirations, notamment de liquides biologiques, par exemple le sang ou d'autres liquides biologiques, au sein des bâtiments hospitaliers ou analogues.

## Revendications

1. Ensemble de distribution de fluide (1) destiné à être fixé à une paroi d'un bâtiment hospitalier comprenant une prise de distribution de fluide (2) comprenant un corps de prise (3) et un boitier (20) agencé autour du corps de prise (3) de la prise de distribution de fluide (2), ledit boitier (20) comprenant un demi-boitier avant (20A) et une façade avant (21) comprenant une ouverture centrale (24) donnant accès à la prise de distribution de fluide (2), et une pièce-écrou (40) comprenant un orifice central (41) et une face extérieure (44) en couronne orientée vers l'extérieur du boitier (20), ladite pièce-écrou (40) étant fixée au corps de prise (3) de sorte que la façade avant (21) soit prise en sandwich et maintenue entre la pièce-écrou (40) et le corps de prise (3), **caractérisé en ce que** :
- le demi-boitier avant (20A) formant le corps principal du boitier rigide (20) est situé entre la face avant (21) et une pièce-étrier (10), ledit demi boitier (20A) étant pris en 'sandwich' entre la face avant (21) et la paroi du bâtiment hospitalier, et ledit corps de prise (3) étant maintenu sur la paroi du bâtiment hospitalier, par la pièce-étrier (10),
- plusieurs logements (43) sont agencés dans la face extérieure (44) en couronne de la pièce-écrou (40), lesdits logements (43) étant dimensionnés pour recevoir un outil spécifique de montage/démontage de la pièce-écrou (40),
- la pièce-écrou (40) comprend en outre un orifice central (41) comprenant un taraudage (42) aménagé dans la paroi périphérique délimitant l'orifice central (41), ledit taraudage (42) de la pièce-écrou (40) coopérant avec un filetage périphérique porté par le corps de prise (3) de la prise de distribution de fluide (2) pour fixer par vissage la pièce-écrou (40) audit corps de prise (3) au moyen dudit outil spécifique de montage/démontage, et
- la prise de distribution de fluide (2) comporte en outre un guide-embout (8) agencé coaxialement dans le corps de prise (3), ledit guide-embout (8) comprenant une tête de raccordement (8A) faisant saillie axialement vers l'extérieur au travers de l'ouverture centrale (24) de la façade (21) du boitier (20), l'orifice central (41) de la pièce-écrou (40) étant traversé par la tête de raccordement (8A) du guide-embout (8).

2. Ensemble selon la revendication 1, **caractérisé en ce que** les logements (43) aménagés dans la face extérieure (44) en couronne de la pièce-écrou (40) sont borgnes.

3. Ensemble selon la revendication 1, **caractérisé en ce que** le taraudage (42) est aménagé dans l'épaisseur de la pièce-écrou (40) au sein de l'orifice central (41).

4. Ensemble selon la revendication 1, **caractérisé en ce que** la pièce-étrier (10) comprend une ouverture (11) bordée par un rebord arqué venant se loger dans une gorge annulaire (7) du corps de prise (3).

5. Ensemble selon l'une des revendications précédentes, **caractérisé en ce qu'**une rainure (25) annulaire aménagée autour de l'ouverture centrale (24) de la façade (21) du boitier (20) de manière à former une zone en retrait (24-25), de préférence en forme de disque.

6. Ensemble selon la revendication 5, **caractérisé en ce que** la pièce-écrou (40) vient se loger dans la zone en retrait (24-25) en forme de disque lorsque la pièce-écrou (40) maintient la façade avant (21) au contact du corps de prise (3),

7. Ensemble selon l'une des revendications précédentes, **caractérisé en ce que** le boitier (20) a une forme parallélépipédique.

8. Ensemble selon l'une des revendications précédentes, **caractérisé en ce que** le boitier (20) comprend le demi-boitier avant (20A) et un demi-boitier arrière (20B) fixés l'un à l'autre.

9. Ensemble selon l'une des revendications précédentes, **caractérisé en ce qu'**une tubulure (9) verticale est connectée fluidiquement au corps (3) de la prise (2).

10. Ensemble selon l'une des revendications précédentes, **caractérisé en ce que** le boitier (20) comprend en outre un couvercle (22) pivotant porté par la façade avant (21).

11. Ensemble selon l'une des revendications précédentes, **caractérisé en ce que** le boitier (20) comprend en outre une ouverture de toit (27), située sur le dessus du boitier (20), traversée par la tubulure (9) verticale connectée fluidiquement au corps (3) de la prise (2).

12. Ensemble selon l'une des revendications précédentes, **caractérisé en ce qu'**il comprend en outre une clé spécifique de montage/démontage comprenant des griffes aptes à venir s'insérer dans les logements (43) de la pièce-écrou (40) afin de permettre à un opérateur d'exercer un couple dans le sens horaire ou antihoraire afin de visser ou dévisser la pièce-écrou (40).

13. Ensemble selon la revendication 1, **caractérisé en ce que** l'orifice central (41) de la pièce-écrou (40), le corps de prise (3) et l'ouverture centrale (24) de la façade avant (21) sont coaxiaux (axe AA).

14. Ensemble selon la revendication 9, **caractérisé en ce que** la tubulure (9) verticale est raccordé fluidiquement au réseau de fluide d'un bâtiment hospitalier.

## Patentansprüche

1. Fluidabgabeanordnung (1), welche dazu bestimmt ist, an einer Wand eines Krankenhausgebäudes befestigt zu werden, und einen Fluidabgabeanschluss (2) umfasst, der einen Anschlusskörper (3) und ein Gehäuse (20), das um den Anschlusskörper (3) des Fluidabgabeanschlusses (2) herum angeordnet ist, umfasst, wobei das Gehäuse (20) eine vordere Gehäusehälfte (20A) und eine vordere Frontwand (21), die eine den Zugang zu dem Fluidabgabeanschluss (2) ermöglichende zentrale Öffnung (24) umfasst, umfasst, und ein Mutternstück (40), das ein zentrales Durchgangsloch (41) und eine kranzförmige Außenfläche (44), die der Außenseite des Gehäuses (20) zugewandt ist, umfasst, wobei das Mutternstück (40) an dem Anschlusskörper (3) so befestigt ist, dass die vordere Frontwand (21) zwischen dem Mutternstück (40) und dem Anschlusskörper (3) sandwichartig angeordnet ist und gehalten wird, **dadurch gekennzeichnet, dass**:
- die vordere Gehäusehälfte (20A), die den Hauptkörper des starren Gehäuses (20) bildet, sich zwischen der vorderen Frontwand (21) und einem Bügelstück (10) befindet, wobei die Gehäusehälfte (20A) sandwichartig zwischen der vorderen Frontwand (21) und der Wand des Krankenhausgebäudes angeordnet ist und der Anschlusskörper (3) durch das Bügelstück (10) an der Wand des Krankenhausgebäudes gehalten wird,
- mehrere Aufnahmen (43) in der kranzförmigen Außenfläche (44) des Mutternstücks (40) angeordnet sind, wobei die Aufnahmen (43) dafür bemessen sind, ein spezielles Werkzeug für die Montage/Demontage des Mutternstücks (40) aufzunehmen,
- das Mutternstück (40) außerdem ein zentrales Durchgangsloch (41) umfasst, das ein Innengewinde (42) umfasst, das in der das zentrale Durchgangsloch (41) begrenzenden Umfangswand angeordnet ist, wobei das Innengewinde (42) des Mutternstücks (40) mit einem von dem Anschlusskörper (3) des Fluidabgabeanschlusses (2) getragenen Umfangsgewinde zusammenwirkt, um mittels des speziellen Montage-/Demontagewerkzeugs das Mutternstück (40) durch Verschraubung an dem Anschlusskörper (3) zu befestigen, und
- der Fluidabgabeanschluss (2) außerdem eine Endstückführung (8) aufweist, die im Anschlusskörper (3) koaxial angeordnet ist, wobei die Endstückführung (8) einen Anschlusskopf (8A) umfasst, der durch die zentrale Öffnung (24) der Frontwand (21) des Gehäuses (20) hindurch axial nach außen vorsteht, wobei das das zentrale Durchgangsloch (41) des Mutternstücks (40) von dem Anschlusskopf (8A) der Endstückführung (8) durchquert wird.

2. Anordnung nach Anspruch 1, **dadurch gekennzeichnet, dass** die Aufnahmen (43), die in der kranzförmigen Außenfläche (44) des Mutternstücks (40) angeordnet sind, nicht durchgehend sind.

3. Anordnung nach Anspruch 1, **dadurch gekennzeichnet, dass** das Innengewinde (42) in der Dicke des Mutternstücks (40) innerhalb des zentralen Durchgangslochs (41) angeordnet ist.

4. Anordnung nach Anspruch 1, **dadurch gekennzeichnet, dass** das Bügelstück (10) eine Öffnung (11) umfasst, die von einer bogenförmigen Randleiste umrandet wird, die in einer Ringnut (7) des Anschlusskörpers (3) aufgenommen wird.

5. Anordnung nach einem der vorhergehenden Ansprüche, **dadurch gekennzeichnet, dass** eine Ringnut (25) um die zentrale Öffnung (24) der Frontwand (21) des Gehäuses (20) herum so angeordnet ist, dass ein zurückspringender Bereich (24-25) gebildet wird, der vorzugsweise scheibenförmig ist.

6. Anordnung nach Anspruch 5, **dadurch gekennzeichnet, dass** das Mutternstück (40) in dem scheibenförmigen zurückspringenden Bereich (24-25) aufgenommen wird, wenn das Mutternstück (40) die vordere Frontwand (21) in Kontakt mit dem Anschlusskörper (3) hält.

7. Anordnung nach einem der vorhergehenden Ansprüche, **dadurch gekennzeichnet, dass** das Gehäuse (20) eine Quaderform aufweist.

8. Anordnung nach einem der vorhergehenden Ansprüche, **dadurch gekennzeichnet, dass** das Gehäuse (20) die vordere Gehäusehälfte (20A) und eine hintere Gehäusehälfte (20B) umfasst, die aneinander befestigt sind.

9. Anordnung nach einem der vorhergehenden Ansprüche, **dadurch gekennzeichnet, dass** ein vertikaler Stutzen (9) mit dem Körper (3) des Anschlusses (2) fluidisch verbunden ist.

10. Anordnung nach einem der vorhergehenden Ansprüche, **dadurch gekennzeichnet, dass** das Gehäuse (20) außerdem einen schwenkbaren Deckel (22) umfasst, der von der vorderen Frontwand (21) getragen wird.

11. Anordnung nach einem der vorhergehenden Ansprüche, **dadurch gekennzeichnet, dass** das Gehäuse (20) außerdem eine Dachöffnung (27) umfasst, die sich auf der Oberseite des Gehäuses (20) befindet und von dem vertikalen Stutzen (9) durchquert wird, der mit dem Körper (3) des Anschlusses (2) fluidisch verbunden ist.

12. Anordnung nach einem der vorhergehenden Ansprüche, **dadurch gekennzeichnet, dass** sie außerdem einen speziellen Montage-/Demontageschlüssel umfasst, der Klauen umfasst, die geeignet sind, in die Aufnahmen (43) des Mutternstücks (40) eingesetzt zu werden, um einer Bedienperson zu ermöglichen, ein Drehmoment im Uhrzeigersinn oder entgegen dem Uhrzeigersinn auszuüben, um das Mutternstück (40) anzuschrauben oder loszuschrauben.

13. Anordnung nach Anspruch 1, **dadurch gekennzeichnet, dass** das zentrale Durchgangsloch (41) des Mutternstücks (40), der Anschlusskörper (3) und die zentrale Öffnung (24) der vorderen Frontwand (21) koaxial sind (Achse AA).

14. Anordnung nach Anspruch 9, **dadurch gekennzeichnet, dass** der vertikale Stutzen (9) an das Fluidnetz eines Krankenhausgebäudes fluidisch angeschlossen ist.

## Claims

1. Fluid distribution assembly (1) to be fixed to a wall of a hospital building, comprising a fluid distribution socket (2) comprising a socket body (3) and a box (20) arranged around the socket body (3) of the fluid distribution socket (2), said box (20) comprising a front half-box (20A) and a front panel (21) comprising a central opening (24) giving access to the fluid distribution socket (2), and a nut piece (40) comprising a central orifice (41) and a ring-shaped outer face (44) oriented towards the outside of the box (20), said nut piece (40) being fixed to the socket body (3) so that the front panel (21) is sandwiched and held between the nut piece (40) and the socket body (3), **characterized in that**:
- the front half-box (20A) forming the main body of the rigid box (20) is situated between the front face (21) and a bracket piece (10), said half-box (20A) being sandwiched between the front face (21) and the wall of the hospital building, and said socket body (3) being held on the wall of the hospital building by the bracket piece (10),
- several seats (43) are arranged in the ring-shaped outer face (44) of the nut piece (40), said seats (43) being dimensioned to receive a specific tool for assembly/disassembly of the nut piece (40),
- the nut piece (40) further comprises a central orifice (41) comprising an internal thread (42) formed in the peripheral wall delimiting the central orifice (41), said internal thread (42) of the nut piece (40) interacting with a peripheral thread carried by the socket body (3) of the fluid distribution socket (2) in order to fix the nut piece (40) by screwing to said socket body (3) by means of said specific assembly/disassembly tool, and
- the fluid distribution socket (2) further comprises an endpiece guide (8) arranged coaxially in the socket body (3), said endpiece guide (8) comprising a connection head (8A) projecting axially outwards through the central opening (24) of the panel (21) of the box (20), the central orifice (41) of the nut piece (40) being passed through by the connection head (8A) of the endpiece guide (8).

2. Assembly according to Claim 1, **characterized in that** the seats (43) formed in the ring-shaped outer face (44) of the nut piece (40) are blind.

3. Assembly according to Claim 1, **characterized in that** the internal thread (42) is formed in the thickness of the nut piece (40) within the central orifice (41).

4. Assembly according to Claim 1, **characterized in that** the bracket piece (10) comprises an opening (11) bordered by an arcuate rim which fits into an annular groove (7) of the socket body (3).

5. Assembly according to any one of the preceding claims, **characterized in that** an annular groove (25) is formed around the central opening (24) of the panel (21) of the box (20) so as to form a recessed region (24-25), which is preferably disc-shaped.

6. Assembly according to Claim 5, **characterized in that** the nut piece (40) fits into the disc-shaped recessed region (24-25) when the nut piece (40) holds the front panel (21) in contact with the socket body (3).

7. Assembly according to any one of the preceding claims, **characterized in that** the box (20) has a parallelepipedal shape.

8. Assembly according to any one of the preceding claims, **characterized in that** the box (20) comprises the front half-box (20A) and a rear half-box (20B) fixed to each other.

9. Assembly according to any one of the preceding claims, **characterized in that** a vertical pipe (9) is fluidically connected to the body (3) of the socket (2).

10. Assembly according to any one of the preceding claims, **characterized in that** the box (20) further comprises a pivoting cover (22) carried by the front panel (21).

11. Assembly according to any one of the preceding claims, **characterized in that** the box (20) further comprises a roof opening (27), which is situated on the top of the box (20) and through which extends the vertical pipe (9) fluidically connected to the body (3) of the socket (2).

12. Assembly according to any one of the preceding claims, **characterized in that** it further comprises a specific assembly/disassembly wrench comprising claws adapted to be inserted into the seats (43) of the nut piece (40) in order to allow an operator to exert a torque in the clockwise or anticlockwise direction in order to screw in or unscrew the nut piece (40).

13. Assembly according to Claim 1, **characterized in that** the central orifice (41) of the nut piece (40), the socket body (3) and the central opening (24) of the front panel (21) are coaxial (axis AA).

14. Assembly according to Claim 9, **characterized in that** the vertical pipe (9) is fluidically connected to the fluid network of a hospital building.
